# EUROPEAN PATENT APPLICATION

(11) **EP 3 281 627 A1**
(43) Date of publication of application: **14.02.2018**
(21) Application number: 16776856.3
(22) Date of filing: 06.04.2016
(51) Int. Cl.: A61K 9/70, A61K 38/18, A61K 8/02, A61M 37/00, A61K 38/00, A61K 8/64, A61K 9/00

(54) **SOLUBLE MICRONEEDLE FOR DELIVERING PROTEINS OR PEPTIDES**

(30) Priority: 06.04.2015 KR 20150048462; 06.04.2015 KR 20150048471; 16.10.2015 KR 20150144873; 13.11.2015 KR 20150159966
(71) Applicant: LG Household & Health Care Ltd., Seoul 03184 (KR)
(72) Inventor: SHIM, Woo-Sun, Daejeon 34114 (KR); LEE, Sun-Hwa, Daejeon 34114 (KR); HWANG, Young-Min, Daejeon 34114 (KR); KANG, Nae-Gyu, Daejeon 34114 (KR)
(74) Representative: Goddar, Heinz J.
(86) International application number: PCT/KR2016/003600
(87) International publication number: WO 2016/163753

(57) **Abstract**

The present invention relates to a skin administration system capable of ensuring the stability of peptides or proteins and enhancing the delivery rate of peptides or proteins through the skin and, more particularly, to a microneedle comprising peptides or proteins.

## Description

### TECHNICAL FIELD

The present application claims priority to Korean Patent Application No. 10-2015-0048462 filed on April 6, 2015, Korean Patent Application No. 10-2015-0159966 filed on November 13, 2015, Korean Patent Application No. 10-2015-0048471 filed on April 6, 2015 and Korean Patent Application No. 10-2015-0144873 filed on October 16, 2015, the disclosures of which are incorporated herein by reference.

The present disclosure relates to a soluble microneedle, more particularly to a system for stably delivering a protein or a peptide to the skin, which is capable of improving the stability of the protein or the peptide.

### BACKGROUND ART

Recently, in order to improve skin conditions (e.g., wrinkles, elasticity, etc.), various proteins and peptides are being developed, including epidermal growth factors (EGF), human growth hormones (hGH), transforming growth factors α and β (TGF-α and -β), fibroblast growth factors 1 and 2 (FGF-1 and -2), keratinocyte growth factors (KGF), hepatocyte growth factors (HGF), platelet-derived growth factors (PDGF), etc. A variety of cosmetic products containing these proteins and peptides are commercially available.

For example, EGFs are substances that help growth of granulation tissues and regeneration of blood vessels during the natural wound healing process in human. Because they can provide anti-aging effect, cosmetic products containing EGFs are being developed recently.

However, particularly because of short biological half-life and uncontinued stability, these growth factors are often denatured when contained in general cosmetic formulations and fail to exert their effects. In order to improve the stability, these proteins are encapsulated using microparticles, liposomes, etc. However, these methods are problematic in that direct absorption into the skin is difficult only with application onto the skin because of the large size of the particles or liposomes (tens to hundreds of nanometers or micrometers or greater).

Secondly, in most cases, it is difficult to achieve the desired effect only by increasing the contents of the proteins for improving skin conditions. That is to say, for the protein ingredients such as epidermal growth factors (EGF), human growth hormones, etc., activity is more important than the content and the desired effect cannot be achieved only by increasing the content if the activity is low.

In addition, for the substances beneficial and useful for the skin to exert their effects in actual products, they should be able to penetrate into the epidermal layer and the dermal layer through the horny layer of the skin and a method for uniformly delivering them to the whole skin is necessary. The existing method of using surfactants, etc. to improve penetrability has disadvantages in that the effect of improving penetrability is insignificant and the skin barrier is weakened.

Drug delivery through the skin is used in various applications in various forms due to its convenience of use. These drugs passing through the skin are mainly intended to be delivered to the systemic circulatory system, but some drugs such as those for treating atopy and cosmetics for skin whitening or wrinkle improvement, etc. are intended to be delivered to the skin itself. Despite this convenience and functionality, there are many problems in delivering drugs through the skin due to the intrinsic structure of the skin and it is not easy to develop the drugs passing through the skin. The horny layer of the skin consists of a brick structure composed of keratin-rich keratinocytes and a mortar structure composed of ceramides fatty acids, waxes, etc. filled between the keratinocytes. Because these structures serve as a barrier, the skin has a very low penetrability to materials. Through diffusion, only small molecules with molecular weights of 500 Da or smaller can be delivered into the skin, and only materials having good lipophilicity can pass through the skin.

Due to this structural characteristic of the skin, efforts are being made to enhance the low lipophilicity of peptides by introducing alkyl chains of predetermined lengths in order to improve their absorption into the skin.

However, because the molecular weights of the peptides are increased, it is difficult to substantially improve their absorption into the skin.

Therefore, the inventors of the present disclosure have researched on a method for effectively delivering various peptides and proteins capable of providing skin-improving effects into the skin.

### DISCLOSURE

### Technical Problem

The present disclosure is directed to providing a protein administration system capable of stably delivering various proteins, particularly growth factors, for improving skin conditions into the skin, a method for preparing the system and a method for administering proteins (particularly growth factors) into the skin using the system.

The present disclosure is also directed to solving the problem of the existing method of delivery of peptides into the skin by improving the lipophilicity of the peptides caused by increased molecular weights.

The present disclosure provides a peptide administration system capable of stably delivering various peptides for improving skin conditions into the skin, a method for preparing the system and a method for administering peptides with low lipophilicity into the skin using the system.

### Technical Solution

In order to solve the problems described above, the present disclosure provides a microneedle containing a protein or a peptide, wherein a material forming the microneedle is soluble in the skin and the protein or the peptide is stably delivered into the skin as the microneedle is dissolved or disintegrated when the microneedle is applied to the skin.

The inventors of the present disclosure have studied various administration systems but it was not easy to conceive a peptide delivery system which is capable of stably delivering a peptide with low lipophilicity even when the molecular weight of the peptide is increased to improve the lipophilicity. After consistent efforts, the inventors of the present disclosure have surprisingly found out that a peptide or a peptide derivative having an alkyl chain at the N-terminal can be effectively delivered into the skin by including the same in a microneedle soluble in the skin.

A polypeptide refers to a chain of many amino acids linked by chemical bonds called peptide bonds. The polypeptide is also called simply a peptide.

In order to solve the problems described above, the microneedle should be soluble in the skin. To prepare the soluble microneedle, a water-soluble polymer such as hyaluronic acid, sodium carboxymethyl cellulose (Na-CMC), a vinylpyrrolidone-vinyl acetate copolymer, polyvinyl alcohol, polyvinylpyrrolidone, etc., a saccharide such as xylose, sucrose, maltose, lactose, trehalose, etc. or a mixture thereof may be used. In particular, a mixture of hyaluronic acid (or oligo-hyaluronic acid), sodium carboxymethyl cellulose (Na-CMC) and a saccharide (more specifically, trehalose) may be used when considering the skin penetrability, dissolution rate in the skin, etc. of the microneedle. More specifically, a mixture further containing glycerin described below may be used. Specifically, the microneedle according to the present disclosure may further contain, in addition to the above-described ingredients forming the microneedle, a plasticizer, a surfactant, a preservative, an anti-inflammatory agent, etc.

As the plasticizer, for example, a polyol such as ethylene glycol, propylene glycol, dipropylene glycol, butylene glycol, glycerin, etc. may be used alone or in combination.

Specifically, the microneedle of the present disclosure comprises the peptide in an amount of 0.01-20 wt%, more specifically 0.1-5 wt%, based on the total weight of a solution for preparing the microneedle.

The peptide that can be used in the present disclosure may be a peptide consisting of 3-10 amino acids. Specifically, it may be a peptide having a C₁₀₋₂₀ alkyl group at the N-terminal. The peptide may have a molecular weight measured by gel permeation chromatography of 200-3,000 Da.

Specifically, the peptide may be one selected from a group consisting of a tripeptide, a tetrapeptide, a pentapeptide, a hexapeptide, a heptapeptide, a palmitoyl tripeptide, a myristoyl tetrapeptide, a caprooyl tetrapeptide, a myristoyl pentapeptide, a palmitoyl pentapeptide, a myristoyl hexapeptide, a palmitoyl hexapeptide, a palmitoyl heptapeptide or a mixture thereof.

For example, the palmitoyl tripeptide may be specifically palmitoyl tripeptide-5 (Pal-Lys-Val-Lys-OH), the myristoyl tetrapeptide may be myristoyl tetrapeptide-12 (Myr-Lys-Ala-Lys-Ala-NH₂), the caprooyl tetrapeptide may be caprooyl tetrapeptide-3 (Cap-Lys-Gly-His-Lys), the myristoyl pentapeptide may be myristoyl pentapeptide-17 (Myr-Lys-Leu-Ala-Lys-Lys-NH₂), the palmitoyl pentapeptide may be palmitoyl pentapeptide-4 (Pal-Lys-Thr-Thr-Lys-Ser-OH), the myristoyl hexapeptide may be myristoyl hexapeptide-16 (Myr-Ala-Asp-Leu-Lys-Pro-Thr), the palmitoyl hexapeptide may be palmitoyl hexapeptide-12 (Pal-Val-Gly-Val-Ala-Pro-Gly) and the palmitoyl heptapeptide may be palmitoyl heptapeptide-18 (Pal-Tyr-Pro-Trp-Gln-Arg-Phe).

The present disclosure also provides a microneedle patch system for administering (delivering) a peptide, having the microneedle attached.

The present disclosure also provides a method for preparing a microneedle containing a peptide or a protein, including: (S1) a step of preparing a solution containing the peptide or the protein and a material soluble in the skin; (S2) a step of injecting the solution into a microneedle mold; and (S3) a step of drying a microneedle and separating the same from the mold.

Specifically, the microneedle may contain a peptide or a peptide derivative having a molecular weight of 200-3000 Da. The present disclosure also provides a method for administering a peptide into the skin with an improved skin permeation efficiency using the microneedle according to the present disclosure.

The present disclosure also provides a use of a microneedle containing a peptide with a large molecular weight for improving wrinkles.

The present disclosure also provides a microneedle containing a microparticle containing a protein or a peptide, wherein a material forming the microneedle is soluble in the skin and the protein or the peptide is stably delivered into the skin as the microneedle is dissolved or disintegrated when the microneedle is applied to the skin.

Because the microparticle contains a polymer forming a hydrophobic core, the protein or the peptide may be stably delivered to the skin.

In the present disclosure, the "protein" or the "peptide" is not necessarily distinguished from each other and is used as a broad concept including an amino acid polymer.

In general, the protein refers to an amino acid polymer having a larger molecular weight than the peptide and a polymer consisting of 50 or less amino acids is known as the peptide. However, in the present disclosure, the protein or the peptide is not necessarily limited by the number of amino acids.

The inventors of the present disclosure have studied various administration systems. After consistent efforts, they have surprisingly found out that a protein or a peptide can be effectively delivered into the skin by impregnating a microparticle containing a protein in a soluble microneedle. When the microparticle entrapping the protein is impregnated in the soluble microneedle and then applied to the skin, the protein is delivered by the microneedle into the skin without pain. The microparticle entrapping the protein is delivered into the skin as the microneedle is dissolved by water in the skin.

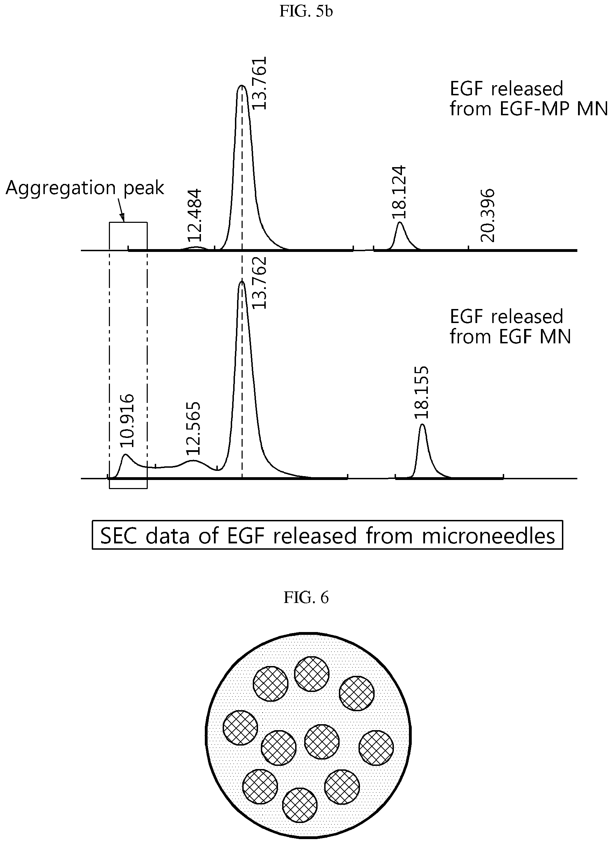

In the present disclosure, the "microparticle" entrapping the "protein" means that the protein is present inside the microparticle in a state completely enclosed by the microparticle. For example, the cross section of the microparticle entrapping the protein may be as shown in FIG. 6, although it is only exemplary.

"Impregnation" means inclusion, including not only the state where the microparticle is present inside the microneedle and completely isolated from the external environment but also the state where the microparticle is partly exposed on the surface of the microneedle. It is to be understood that the "impregnation in the microneedle" embraces not only the state where the microparticle is completely included inside the microneedle but also the state where the microparticle is included in the microneedle such that the microparticle can be administered together with the microneedle when the microneedle is applied to the skin.

The protein, particularly a growth factor, may be effectively delivered into the skin as it is released from the microparticle delivered into the skin. The growth factor used in the present disclosure may include a growth hormone.

In order to achieve the object of the present disclosure, the microneedle should be soluble in the skin. To prepare the soluble microneedle, a water-soluble polymer such as hyaluronic acid, sodium carboxymethyl cellulose (Na-CMC), a vinylpyrrolidone-vinyl acetate copolymer, polyvinyl alcohol, polyvinylpyrrolidone, etc., a saccharide such as xylose, sucrose, maltose, lactose, trehalose, etc. or a mixture thereof may be used. In particular, a mixture of hyaluronic acid (or oligo-hyaluronic acid), sodium carboxymethyl cellulose (Na-CMC) and a saccharide (more specifically, trehalose) may be used when considering the skin penetrability, dissolution rate in the skin, etc. of the microneedle. More specifically, a mixture further containing glycerin may be used. Specifically, the microneedle according to the present disclosure may further contain, in addition to the microparticle containing the protein, particularly a growth factor, and the above-described ingredients forming the microneedle, a plasticizer, a surfactant, a preservative, an anti-inflammatory agent, etc. The plasticizer, the surfactant, the preservative, the anti-inflammatory agent, etc. may include not only those described in the present disclosure but also those commonly used in the art.

In the present disclosure, the material forming the microparticle together with the protein should be stably includable such that the protein is not structurally deformed during the preparation of the microneedle. In particular, the material forming the microparticle should be capable of forming a hydrophobic core so that it can provide stability without structural deformation of the protein.

As the material forming the microparticle, a polymer capable of forming a hydrophobic core may be used. As the polymer, a biodegradable polymer such as polylactide, polyglycolide, poly(lactide-co-glycolide), polyanhydride, polyorthoester, polyetherester, polycaprolactone, monomethoxypolyethylene glycol-polycaprolactone (MPEG-PCL), polyesteramide, polybutyric acid, polyvaleric acid, polyurethane or a copolymer thereof or a non-biodegradable polymer such as polyacrylate, ethylene-vinyl acetate, acryl-substituted cellulose acetate, non-degradable polyurethane, polystyrene, polyvinyl chloride, polyvinyl fluoride, polyvinylimidazole, chlorosulfonated polyolefin, polyethylene oxide or a copolymer thereof may be used alone or in combination, although the present disclosure is not limited thereto.

Specifically, when considering the stable inclusion, releasability in the skin, etc. of the protein, particularly a growth factor, a mixture of one or more of polylactide, polyglycolide and poly(lactide-co-glycolide) with monomethoxypolyethylene glycol-polycaprolactone (MPEG-PCL) may be used as the polymer forming a hydrophobic core.

The microparticle may be either a matrix type or a reservoir type as long as the purpose of the present disclosure can be achieved.

The microparticle that can be used in the present disclosure may be prepared by various methods well known in the art to which the present disclosure belongs. For example, the microparticle that can be used in the present disclosure may be prepared by a solvent exchange method, a solvent evaporation method, a membrane dialysis method, a spray drying method, etc. For example, the methods described in the literatures Journal of Controlled Release, 70(2001), 1-20 and International Journal of PharmTech Research, 3(2011), 1242-1254 may be used. Specifically, it may be prepared by the commonly used emulsification and solvent evaporation method.

Specifically, the microparticle according to the present disclosure may have a diameter of 0.01-10 µm. If the particle size exceeds 10 µm, skin penetration may be difficult because the needle strength is decreased when the microparticle is impregnated in the microneedle. The diameter of the microparticle according to the present disclosure is measured by laser light scattering (LLS). For example, it may be measured using Malvern's Zetasizer 2000™.

Specifically, the microparticle of the present disclosure may contain 0.01-20 wt%, more specifically 0.1-5 wt%, of the protein or the peptide based on the total weight of the microparticle. And, the microneedle of the present disclosure may contain specifically 0.05-10 wt%, more specifically 0.1-5 wt%, of the microparticle based on the total weight of the microneedle.

Specifically, the protein that can be used in the present disclosure may be particularly a growth factor or a growth hormone. The growth factor or the growth hormone is a protein involved in the growth, proliferation and differentiation of cells. Due to the issues of selective tissue or organ compatibility, structural deformation of the protein during delivery, etc., an appropriate system or method for delivery has been demanded. After consistent efforts, the inventors of the present disclosure have found out that application of a microparticle to a microneedle is effective in the delivery of a protein, particularly a growth factor and/or a growth hormone.

The growth factor may be one or more selected from a group consisting of bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor-a and -β (TGF-α, -β), brain-derived neurotrophic factor (BDNF), platelet-derived growth factor (PDGF), placental growth factor (PlGF), hepatocyte growth factor (HGF), fibroblast growth factor 1 and 2 (FGF-1, -2), keratinocyte growth factor (KGF) and an analogue thereof.

The analogue used in the present disclosure may have a sequence homology of 80%, specifically 85%, more specifically 90%, with the proteins.

The present disclosure also provides a microneedle patch system for administering (delivering) a protein with the microneedle attached. Specifically, an exemplary embodiment of the present disclosure provides a method for cosmetically administering a protein or a peptide to the skin.

The present disclosure also provides a method for preparing a microneedle containing a peptide or a protein, which exhibits improved structural stability or aggregation, including: (S1) a step of preparing a solution containing the peptide or the protein and a material soluble in the skin; (S2) a step of injecting the solution into a microneedle mold; and (S3) a step of drying a microneedle and separating the same from the mold, wherein the step (S1) further includes a step of entrapping the peptide or the protein in a microparticle and the step of entrapping the peptide or the protein in a microparticle includes including the peptide or the protein inside the microparticle using a polymer forming a hydrophobic core.

The present disclosure also provides a method for administering a protein to the skin with high skin penetrability and stability by using the microneedle according to the present disclosure.

The present disclosure also provides a use of a microneedle containing a microparticle containing a protein, specifically a growth factor or a growth hormone, more specifically EGF, TGF-β or hGH, for improving wrinkles.

An exemplary embodiment of the present disclosure provides a method for administering a peptide with a molecular weight 200-3000 Da to the skin by attaching a microneedle containing the peptide to the skin.

An exemplary embodiment of the present disclosure provides a use of a microneedle containing a peptide with a molecular weight 200-3000 Da for improving skin wrinkles.

An exemplary embodiment of the present disclosure provides a method for administering a growth factor to the skin by attaching a microneedle containing a microparticle entrapping the growth factor to the skin.

An exemplary embodiment of the present disclosure provides a use of a microneedle containing a microparticle entrapping the growth factor for improving skin wrinkles.

### Advantageous Effects

The present disclosure provides a microneedle which enhances the skin permeation efficiency of a peptide with a large molecular weight.

The present disclosure also provides a microneedle for administering a peptide to the skin with increased skin permeation efficiency. The present disclosure also provides a method for administering a peptide to the skin using the microneedle.

The present disclosure provides a microneedle for administering a protein, particularly a growth factor, to the skin with ensured stability and improved skin permeation. The present disclosure also provides a microneedle for administration to a skin, which is capable of stably delivering a protein to the skin without inducing structural deformation.

The present disclosure provides a system for delivering a protein to the skin, which is capable of stably delivering a protein into the skin without aggregation between proteins that may be induced when the protein is contained in general cosmetic formulations.

The present disclosure also provides a method for administering a growth factor to the skin using the microneedle.

### DESCRIPTION OF DRAWINGS

The drawings attached to the specification illustrate specific exemplary embodiments of the present disclosure and are provided for better understanding of the technical idea of the present disclosure together with the foregoing description. Therefore, the present disclosure should not be construed as being limited to the drawings.

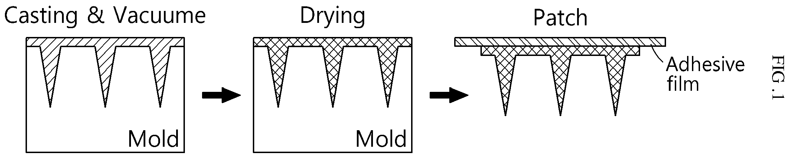

FIG. 1 shows an exemplary embodiment of various methods for preparing a microneedle according to the present disclosure. The soluble microneedle may be prepared by a solution casting method. It may be prepared by casting a solution in a mold, applying vacuum and/or centrifugal force to fill the solution in the hollow cavity of the mold, and then drying the solution. As a material for forming the microneedle, a commonly used synthetic or natural water-soluble polymer may be used.
FIG. 2 shows a Franz diffusion cell for testing the release behavior of a drug contained in a microneedle according to the present disclosure.
FIG. 3 shows a result of measuring release of EGF from a microneedle using pig skin loaded in a Franz diffusion cell.
FIG. 4 shows a result of measuring improvement of eye wrinkles after long-term use of an EGF solution-impregnated microneedle (EGF MN) and an EGF microparticle-impregnated microneedle (EGF-MP MN) according to the present disclosure.
FIGS. 5a and 5b show a result of analyzing the stability of EGF by size exclusion chromatography (SEC). FIG. 5a shows the SEC data of EGF standard and FIG. 5b shows the SEC data of EGF released from microneedles.
FIG. 6 schematically shows a microparticle according to an exemplary embodiment.
FIG. 7 shows a result of measuring release of a peptide from a microneedle using pig skin loaded in a Franz diffusion cell.

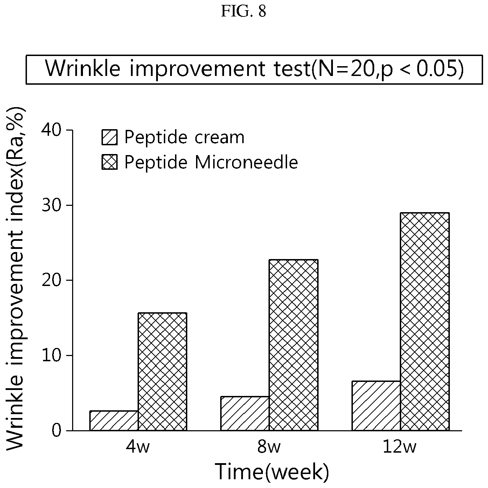

FIG. 8 shows a result of measuring improvement of eye wrinkles after long-term use of a peptide cream and a peptide-impregnated microneedle (Peptide Microneedle) according to the present disclosure.

### MODE FOR DISCLOSURE

Hereinafter, the present disclosure is described in detail through examples in order to help understanding. However, the examples according to the present disclosure can be modified into various different forms and the scope of the present disclosure should not be construed as being limited to the following examples. The examples of the present disclosure are provided to fully explain the present disclosure to those of ordinary skill in the related art.

### <Preparation of protein-loaded microparticle>

1 g of poly(lactic-co-glycolic acid) (PLGA) was dissolved in 10 mL of methylene chloride. Then, a W/O emulsion was prepared by slowly adding an aqueous solution of 200 mg of a polypeptide (epidermal growth factor, EGF) dissolved in 2 mL of purified water to the PLGA solution. To a 0.2% polyvinyl alcohol aqueous solution (100 mL), the prepared W/O emulsion solution was added with stirring. The organic solvent methylene chloride was evaporated from the prepared W/O/W double emulsion by stirring at room temperature for 24 hours to obtain an EGF-loaded microparticle. The remaining organic solvent and water were removed using a rotary evaporator so that the content of EGF was 0.2%. As a result of analysis using the ELISA kit, the EGF content was 0.21%. And, the average size of the microparticle was analyzed to be 350 nm by a particle size analyzer.

### <Preparation of EGF microparticle-loaded microneedle>

An EGF (in solution state)- or EGF microparticle-loaded soluble microneedle was prepared as described in Table 1. In Table 1, the contents are presented in wt% unit.

**[Table 1]**

| Ingredients | EGF MN (wt%) | EGF-MP MN (wt%) |
|---|---|---|
| Oligo- HA | 6 | 6 |
| Na-CMC | 6 | 6 |
| Trehalose | 10 | 10 |
| Glycerin | 5 | 5 |
| HCO-40 | 0.2 | 0.2 |
| EGF | 0.05 | - |
| EGF microparticle (0.2%) | - | 25 |
| Water | To 100 | To 100 |

Specifically, an EGF-loaded soluble microneedle was prepared as follows. After dissolving oligo-HA (hyaluronic acid), Na-CMC (sodium carboxymethyl cellulose) and trehalose in purified water, glycerin, HCO-40 and EGF were added to prepare an EGF solution. The prepared EGF solution was cast in a silicone microneedle mold and then filled in the hollow cavity of the mold by centrifuging at 3000 rpm for 10 minutes. After the filling, the solution was dried in an oven at 70 °C for 3 hours and the resulting microneedle was separated from the silicone mold using an adhesive film.

Specifically, an EGF microparticle (EGF-MP)-loaded soluble microneedle was prepared as follows. After dissolving oligo-HA (hyaluronic acid), Na-CMC (sodium carboxymethyl cellulose) and trehalose in purified water, glycerin, HCO-40 and an EGF microparticle (EGF 0.2%) were added to prepare a solution. The prepared solution was cast in a silicone microneedle mold and then filled in the hollow cavity of the mold by centrifuging at 3000 rpm for 10 minutes. After the filling, the solution was dried in an oven at 70 °C for 3 hours and the resulting microneedle was separated from the silicone mold using an adhesive film.

### <Oil-in-water EGF cream>

For comparison of skin penetration with EGF loaded in the microneedle, EGF was loaded in a commonly used oil-in-water cream formulation as described in Table 2. The contents are presented in wt% unit.

**[Table 2]**

| Ingredients | Contents (wt%) |
|---|---|
| C₁₄₋₂₂ alcohol and C₁₂₋₂₀ alkyl glucoside(mixture C₁₄₋₂₂ alcohol:C₁₂₋₂₀ alkyl glucoside = 80:20, w/w) Glyceryl stearate and PEG-100 stearate(mixture 50:50, w/w) Glyceryl stearate Cetearyl alcohol Polyglyceryl-3 methylglucose distearate Hydrogenated polydecene Cyclohexasiloxane Carbomer Tromethamine Glycerin Dipropylene glycol 1,2-Hexanediol EGF (epidermal growth factor) Purified water | 1.51.20.91.51.54. 53.50.20.23520.0 5 Balance (to 100) |

### <Drug release behavior>

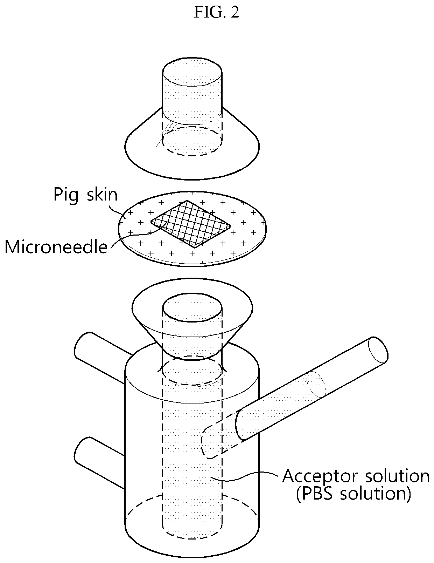

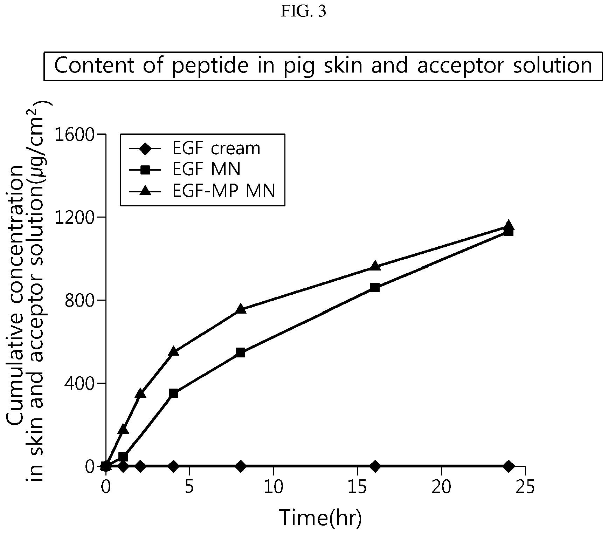

The release of EGF from the microneedle prepared above was tested using pig skin loaded in a Franz diffusion cell (see FIG. 2). PBS containing 30 wt% DPG was used as an acceptor solution. The EGF content in the pig skin tissue and in the acceptor solution with time was measured using the Franz diffusion cell and the ELISA kit. After applying the EGF cream on the pig skin or attaching the EGF- or EGF-MP-loaded microneedle, the penetration amount of EGF into the skin with time was investigated. The microneedle was infiltrated into the pig skin and removed after being dissolved (2 hours, 32 °C). Then, the pig skin to which EGF was delivered by the microneedle was loaded in a Franz diffusion cell and the release behavior of EGF from the pig skin to the acceptor solution was observed with time. The result is shown in FIG. 3.

As seen from FIG. 3, the skin penetration amount was about 500 times or more, with 1 µg or more, for the EGF- and EGF-MP-loaded microneedles as compared to the cream because EGF was delivery directly into the skin by the microneedles.

### <Improvement of wrinkles>

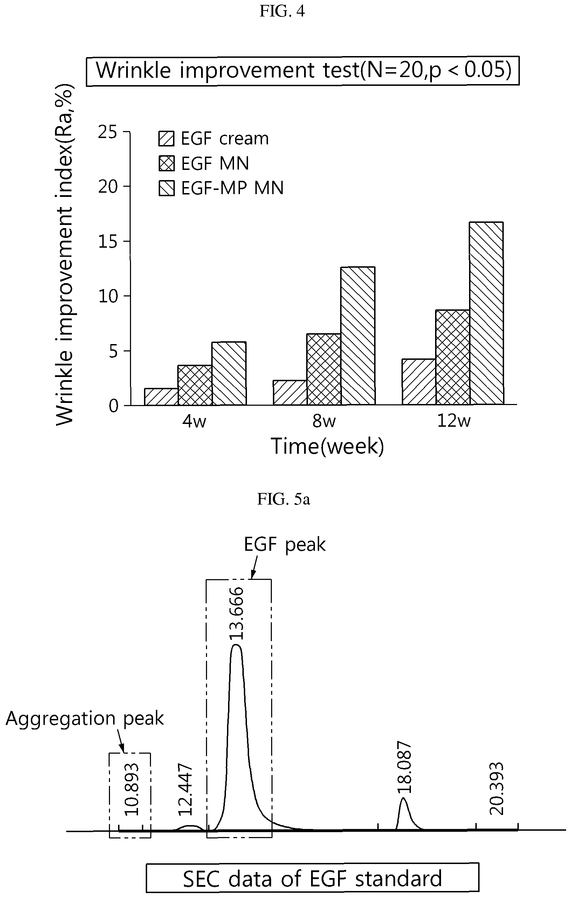

After treating the EGF cream, the EGF-loaded microneedle and the EGF microparticle-loaded microneedle on eye wrinkles every day for 12 weeks, the degree of wrinkle improvement was evaluated by silicone replica image analysis (N = 20). The result is shown in FIG. 4. The EGF-loaded microneedles showed better improvement than the EGF cream and the EGF-MP-loaded microneedle showed excellent effect of improving wrinkles, suggesting that EGF is effectively delivered into the skin by the EGF-MP-loaded microneedle. It is because EGF is released from the EGF-MP delivered into the skin with a stable structure.

### <Analysis of EGF stability (SEC)>

It was investigated by size exclusion chromatography (SEC) whether the structure of EGF was deformed when it was released from the microneedle.

When the EGF itself was loaded in the microneedle and delivered into the skin, the aggregation peak was increased relatively due to the aggregation and structural deformation of EGF. In contrast, when it was loaded in the microneedle after being stably entrapped in the microparticle, aggregation did not occur after delivery into the skin and a result similar to that of EGF standard was obtained.

Accordingly, it can be seen that a polypeptide or a protein such as EGF can be delivered into the skin with high efficiency and stability if it is stably entrapped in a microparticle and then loaded in a microneedle.

### <Preparation of peptide microneedle>

A peptide (in solution state)-loaded soluble microneedle was prepared as described in Table 3. In Table 3, the contents are presented in wt% unit.

**[Table 3]**

| Ingredients | Peptide MN (wt%) |
|---|---|
| Oligo- HA | 6 |
| Na-CMC | 6 |
| Trehalose | 10 |
| Glycerin | 5 |
| HCO-40 | 0.2 |
| Genistein | - |
| Peptide (myristoyl tetrapeptide-6)-DPG solution (10%) | 1.0 |
| Water | To 100 |

Specifically, a peptide (myristoyl tetrapeptide-6)-loaded soluble microneedle was prepared as follows.

After dissolving oligo-HA (hyaluronic acid), Na-CMC (sodium carboxymethyl cellulose) and trehalose in purified water, glycerin, HCO-40 and a peptide solution (peptide 10%, DPG 90%) were added to prepare a solution in which the peptide is dispersed (DPG: dipropylene glycol). The prepared peptide dispersion was cast in a silicone microneedle mold and then filled in the hollow cavity of the mold by centrifuging at 3000 rpm for 10 minutes. After the filling, the solution was dried in an oven at 70 °C for 3 hours and the resulting microneedle was separated from the silicone mold using an adhesive film.

### <Oil-in-water peptide cream>

For comparison of skin penetration with the peptide loaded in the microneedle, the peptide was loaded in a commonly used oil-in-water cream formulation as described in Table 4. The contents are presented in wt% unit.

**[Table 4]**

| Ingredients | Contents (wt%) |
|---|---|
| C₁₄₋₂₂ alcohol and C₁₂₋₂₀ alkyl glucoside(mixture C₁₄₋₂₂ alcohol:C₁₂₋₂₀ alkyl glucoside = 80:20, w/w)Glyceryl stearate and PEG-100 stearate(mixture 50:50, w/w) Glyceryl stearate | 1.51.20.91.51.54.5 3.50.20.23520.5 Balance (to 100) |
| Cetearyl alcohol Polyglyceryl-3 methylglucose distearate Hydrogenated polydecene Cyclohexasiloxane Carbomer | |
| Tromethamine Glycerin Dipropylene glycol 1,2-Hexanediol Peptide (myristoyl tetrapeptide-6) Purified water | |

### <Drug release behavior>

The release of the peptide from the microneedle prepared above was tested using pig skin loaded in a Franz diffusion cell (see FIG. 2). PBS containing 30 wt% DPG was used as an acceptor solution.

The peptide content in the pig skin tissue and in the acceptor solution with time was measured by liquid chromatography using the Franz diffusion cell.

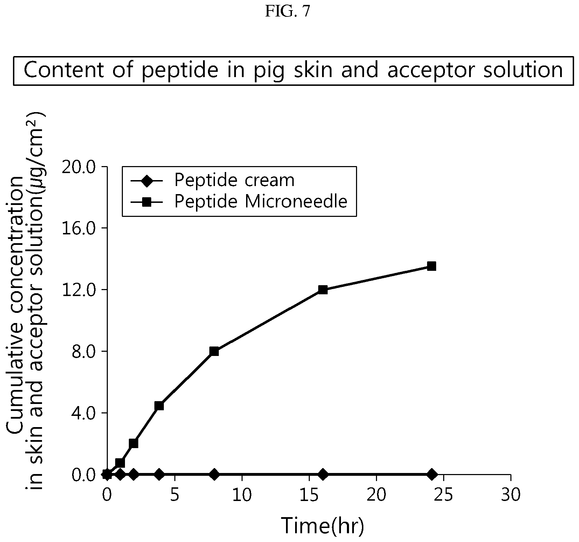

After applying the peptide cream on the pig skin or attaching the peptide-loaded microneedle, the penetration amount of the peptide into the skin with time was investigated. The microneedle was infiltrated into the pig skin and removed after being dissolved (2 hours, 32 °C). Then, the pig skin to which the peptide was delivered by the microneedle was loaded in a Franz diffusion cell and the release behavior of the peptide from the pig skin to the acceptor solution was observed with time. The result is shown in FIG. 7.

As seen from FIG. 7, the skin penetration amount was about 100 times or more, with 15 µg or more, for the peptide-loaded microneedles as compared to the cream because the peptide was delivery directly into the skin by the microneedles.

### <Improvement of wrinkles>

After treating the peptide cream and the peptide-loaded microneedle on eye wrinkles every day for 12 weeks, the degree of wrinkle improvement was evaluated by silicone replica image analysis (N = 20).

The peptide-loaded microneedles showed 5 times or better improvement than the peptide cream. It is because the peptide is effectively delivered into the skin by the microneedle.

### INDUSTRIAL APPLICABILITY

The present disclosure can be used in cosmetic and pharmaceutical applications for improving skin wrinkles.

The microneedle of the present disclosure may provide a superior effect of reducing skin wrinkles.

## Claims

1. A microneedle comprising a peptide or a protein.

2. The microneedle according to claim 1, wherein the peptide is a peptide having a C₁₀₋₂₀ alkyl group at the N-terminal.

3. The microneedle according to claim 1, wherein the peptide has a molecular weight of 200-3000 Da.

4. The microneedle according to claim 1, wherein the peptide is one selected from a group consisting of a tripeptide, a tetrapeptide, a pentapeptide, a hexapeptide, a heptapeptide, a palmitoyl tripeptide, a myristoyl tetrapeptide, a caprooyl tetrapeptide, a myristoyl pentapeptide, a palmitoyl pentapeptide, a myristoyl hexapeptide, a palmitoyl hexapeptide, a palmitoyl heptapeptide or a mixture thereof.

5. The microneedle according to claim 1, wherein a material forming the microneedle is soluble in the skin.

6. The microneedle according to claim 5, wherein the material forming the microneedle is hyaluronic acid, sodium carboxymethyl cellulose (Na-CMC), a vinylpyrrolidone-vinyl acetate copolymer, polyvinyl alcohol, polyvinylpyrrolidone, a saccharide or a mixture thereof.

7. The microneedle according to claim 5, wherein the microneedle further comprises a plasticizer in addition to the material forming the microneedle.

8. The microneedle according to claim 1, wherein the microneedle comprises a microparticle and the peptide or the protein is entrapped inside the microparticle.

9. The microneedle according to claim 8, wherein the material forming the microneedle is soluble in the skin and the microparticle comprises a polymer forming a hydrophobic core.

10. The microneedle according to claim 9, wherein the material forming the microneedle is hyaluronic acid, sodium carboxymethyl cellulose (Na-CMC), a vinylpyrrolidone-vinyl acetate copolymer, polyvinyl alcohol, polyvinylpyrrolidone, a saccharide or a mixture thereof.

11. The microneedle according to claim 9, wherein the polymer forming a hydrophobic core is one or more selected from a group consisting of one or more biodegradable polymer selected from a group consisting of polylactide, polyglycolide, poly(lactide-co-glycolide), polyanhydride, polyorthoester, polyetherester, polycaprolactone, monomethoxypolyethylene glycol-polycaprolactone (MPEG-PCL), polyesteramide, polybutyric acid, polyvaleric acid, polyurethane or a copolymer thereof; and one or more non-biodegradable polymer selected from a group consisting of polyacrylate, ethylene-vinyl acetate, acryl-substituted cellulose acetate, non-degradable polyurethane, polystyrene, polyvinyl chloride, polyvinyl fluoride, polyvinylimidazole, chlorosulfonated polyolefin, polyethylene oxide or a copolymer thereof.

12. The microneedle according to claim 11, wherein the polymer forming a hydrophobic core is a mixture of one or more of polylactide, polyglycolide and poly(lactide-co-glycolide) with monomethoxypolyethylene glycol-polycaprolactone (MPEG-PCL).

13. The microneedle according to claim 8, wherein the microparticle is a matrix type or a reservoir type.

14. The microneedle according to claim 8, wherein the microparticle has a diameter of 0.01-10 µm.

15. The microneedle according to claim 8, wherein the protein entrapped in the microparticle is a growth factor.

16. The microneedle according to claim 15, wherein the growth factor is one or more selected from a group consisting of bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor-a and -β (TGF-α, -β), brain-derived neurotrophic factor (BDNF), platelet-derived growth factor (PDGF), placental growth factor (PlGF), hepatocyte growth factor (HGF), fibroblast growth factor 1 and 2 (FGF-1, -2), keratinocyte growth factor (KGF) and a mixture thereof.

17. The microneedle according to claim 16, wherein the growth factor is epidermal growth factor (EGF).

18. A method for preparing a microneedle comprising a peptide or a protein, comprising:
(S1) a step of preparing a solution comprising the peptide or the protein and a material soluble in the skin;
(S2) a step of injecting the solution into a microneedle mold; and
(S3) a step of drying a microneedle and separating the same from the mold.

19. The method for preparing a microneedle comprising a peptide or a protein according to claim 18, wherein the material soluble in the skin is hyaluronic acid, sodium carboxymethyl cellulose (Na-CMC), a vinylpyrrolidone-vinyl acetate copolymer, polyvinyl alcohol, polyvinylpyrrolidone, a saccharide or a mixture thereof.

20. The method for preparing a microneedle comprising a peptide or a protein according to claim 18, wherein the peptide is one selected from a group consisting of a tripeptide, a tetrapeptide, a pentapeptide, a hexapeptide, a heptapeptide, a palmitoyl tripeptide, a myristoyl tetrapeptide, a caprooyl tetrapeptide, a myristoyl pentapeptide, a palmitoyl pentapeptide, a myristoyl hexapeptide, a palmitoyl hexapeptide, a palmitoyl heptapeptide or a mixture thereof.

21. The method for preparing a microneedle comprising a peptide or a protein according to claim 18, wherein the step (S1) further comprises a step of entrapping the peptide or the protein in a microparticle and the step of entrapping the peptide or the protein in a microparticle comprises including the peptide or the protein inside the microparticle using a polymer forming a hydrophobic core.

22. The method for preparing a microneedle comprising a peptide or a protein according to claim 21, wherein the polymer forming a hydrophobic core is one or more selected from a group consisting of one or more biodegradable polymer selected from a group consisting of polylactide, polyglycolide, poly(lactide-co-glycolide), polyanhydride, polyorthoester, polyetherester, polycaprolactone, monomethoxypolyethylene glycol-polycaprolactone (MPEG-PCL), polyesteramide, polybutyric acid, polyvaleric acid, polyurethane or a copolymer thereof; and one or more non-biodegradable polymer selected from a group consisting of polyacrylate, ethylene-vinyl acetate, acryl-substituted cellulose acetate, non-degradable polyurethane, polystyrene, polyvinyl chloride, polyvinyl fluoride, polyvinylimidazole, chlorosulfonated polyolefin, polyethylene oxide or a copolymer thereof.

23. A method for administering a peptide with a molecular weight of 200-3000 Da to the skin by attaching a microneedle comprising the peptide to the skin.

24. A use of a microneedle comprising a peptide with a molecular weight of 200-3000 Da for improving skin wrinkles.

25. A method for administering a growth factor to the skin by attaching a microneedle comprising a microparticle entrapping the growth factor to the skin.

26. A use of a microneedle comprising a microparticle entrapping a growth factor for improving skin wrinkles.
